# EUROPEAN PATENT APPLICATION

(11) **EP 2 666 786 A1**
(43) Date of publication of application: **27.11.2013**
(21) Application number: 12003960.7
(22) Date of filing: 21.05.2012
(51) Int. Cl.: C07K 16/28

(54) **Immunotherapy for intracranial hemorrhage**

(71) Applicant: PAION Deutschland GmbH, 52062 Aachen (DE); Inserm, 75013 Paris (FR)
(72) Inventor: The designation of the inventor has not yet been filed
(74) Representative: von Renesse, Dorothea

(57) **Abstract**

A binding protein for the treatment of intracranial hemorrhage, wherein the binding protein is capable of binding to the N-terminal domain of the NMDA receptor subunit NR1 (ATD NR1) thereby preventing the t-PA induced proteolysis of the NR1 subunit.

## Description

Intracranial hemorrhage is a blood loss within the skull. Intracranial hemorrhage occurs when a blood vessel within the skull is ruptured or leaks. It can result from physical trauma (as occurs in head injury) or nontraumatic causes (as occurs in hemorrhagic stroke) such as a ruptured aneurysm. Anticoagulant therapy, as well as disorders with blood clotting can heighten the risk that an intracranial hemorrhage will occur.

Intracranial hemorrhage is a serious medical emergency because the accumulation of blood within the skull will lead to increases in intracranial pressure, which can directly crush delicate brain tissue or limit its blood supply, both effects potentially leading to loss of function and integrity with a high rate of mortality. Moreover, severe increases in intracranial pressure can cause potentially deadly brain herniation, in which parts of the brain are squeezed past structures in the skull.

Types of intracranial hemorrhage are roughly grouped into intra-axial and extra-axial. The hemorrhage is considered a focal brain injury; that is, it occurs in a localized spot rather than causing diffuse damage over a wider area.

Intra-axial hemorrhage also known as intracerebral hemorrhage (ICH) is bleeding within the brain itself. This category includes intraparenchymal hemorrhage, or bleeding within the brain tissue, and intraventricular hemorrhage, bleeding within the brain's ventricles (particularly of premature infants). Intra-axial hemorrhages are more dangerous and harder to treat than extra-axial bleeds.

Extra-axial hemorrhage, bleeding that occurs within the skull but outside of the brain tissue, falls into three subtypes, epidural hemorrhage which occurs between the dura mater (the outermost meninx) and the skull, subdural hemorrhage resulting from tearing of the bridging veins in the subdural space (space between the dura mater and the adjacent arachnoid mater) and subarachnoid hemorrhage, which occurs between the arachnoid and pia meningeal layers.

The most prominent form of intracranial hemorrhage is the intracerebral hemorrhage (ICH). ICH denotes direct bleeding into the brain tissue, forming a gradually enlarging hematoma (pooling of blood) within the tissue. It generally occurs in small arteries or arterioles and is commonly due to hypertension, trauma, bleeding disorders, amyloid angiopathy, illicit drug use, and vascular malformations. The hematoma enlarges until its surround pressure limits its growth, or until it decompresses by emptying into the ventricular system, cerebrospinal fluid (CSF) or the pial surface. One third of intracerebral bleed is directed into the brain's ventricles (intraventricular hemorrhage (IVH)). ICH has a mortality rate of 44 percent after 30 days, higher than ischemic stroke or even the highly dangerous subarachnoid hemorrhage.

Another serious, life-threatening type of hemorrhage is subarachnoid hemorrhage (SAH). SAH is defined as bleeding into the cerebrospinal fluid of the subarachnoid space surrounding the brain. The two most common causes of SAH are rupture of aneurysms at the base of the brain and bleeding from vascular malformations near the pial surface. Bleeding into the CSF from a ruptured aneurysm occurs very quickly, causing a rapid increase of intracranial pressure. The bleeding usually only lasts a few seconds but rebleeding is common. Death or deep coma ensues if the bleeding continues. Hemorrhage from other sources is less abrupt and may continue for a longer period of time. SAH has a 40% mortality over a 30 day period.

Spontaneous intracerebral hemorrhage (ICH) is associated with significant morbidity and mortality. The mechanism of clinical deterioration are poorly understood, but may involve an increase of local tissue pressure as a result of mass effect, as well as the effects of toxic substances released from the hematoma, which may in turn reduce regional blood flow, compromise cell viability and finally cause cerebral ischemia, edema and cellular apoptosis. Part of the pathogenesis is a substantial release of glutamate and endogenous tissue plasminogen activator (t-PA), which, in turn, amplifies glutamate-induced damage. Reduced blood flow also facilitates the development of brain edema and, conversely, brain edema formation may restrict overall cerebral perfusion.

Both brain edema and decrease of cerebral blood flow are hallmarks of brain damage in ICH. Successful prevention of such damage depends upon a better understanding of the interactions between hematoma, intracranial hypertension, cerebral edema and reduction of cerebral blood flow, processes, which hence constitute central targets for future therapeutic avenues.

ICH accounts for about 10 - 15 % of all strokes in North America and Europe, and for about 20 - 30 % in East Asia. In general, ICH is classified according to the anatomic site of hemorrhage. Cortical (lobar) hematomas are distinguished from subcortical sites, such as basal ganglia or brainstem. ICH may occur either with head injury or spontaneously.

Among spontaneous, non-traumatic hemorrhages, a distinction is made between primary and secondary ICH. Primary ICH in adults most commonly results from hypertensive arteriopathies, such as fibrinoid necrosis of lipohyalinosis in the brain. Since hypertensive arteriopathies develop predominantly in the basal perforating arteries, most hypertensive ICHs occur in the putamen, thalamus, pons and dentate nucleus of the cerebellum. In contrast, half of the primary cortical hemorrhages are non-hypertensive in nature and thought to arise from cerebral amyloid angiopathy or small vascular malformations. Causative factors of secondary ICH include brain tumors, anticoagulants and thrombolytic agents. Also recreational drugs and sympathomimetics may precipitate secondary ICH.

Currently, from a clinical viewpoint, ICH is the most undesired type of stroke because mortality and morbidity are particularly high, and effective therapy is largely unavailable. Moreover, the cost of care for patients with ICH is among the highest of all brain disorders. Half of the patients with ICH will die in the hospital after requiring expensive ICU care, and an additional 30% will survive as dependents requiring long-term care and rehabilitation.

The most commonly used surgical treatment for ICH is the surgical clot evacuation (craniotomy). However, its benefits are marginal at best, and its uses remain controversial. A meta-analysis of multiple previous surgical trials has not shown benefit for ICH evacuation over nonsurgical treatment, mostly due to the additional brain injury that may be produced during the transparenchymal approach to the clot.

More recently a minimal-invasive technique featuring stereotactic hematoma puncture, local fibrinolysis and subsequent aspiration of the liquefied hematoma has found interest. The lysis of clot remnants with e.g. rt-PA allows to reduce hematoma volume by 50 to 70 % with minimal associated brain damage. Promising clinical results in nonrandomized studies and experimental data showing positive effects on ischemia and the volume of early perihematomal edema support this therapeutic concept. However, the efficacy and safety of this approach have to be further evaluated in additional future clinical trials.

However, there have been reports of t-PA-mediated neurotoxic brain injury with delayed edema formation. Thus, data suggest that injection of t-PA for clot lysis promotes the development of delayed perihematomal edema, counteracting the beneficial effects of rapid hematoma volume decrease.

There is growing evidence that the interaction between endogenous t-PA and the NMDA receptor is responsible for the neurotoxic effect of t-PA. It has been shown that t-PA binds to and cleaves the amino-terminal domain of the NMDA receptor NR1 subunit at Arg260. In the physiological setting, this cleavage is a regulatory mechanism to enhance NMDA receptor signaling into neurons (Fernandez-Monreal et al., 2004; J.Biol.Chem. 279: 50850 -50856). Thus endogenous t-PA acts a positive neuromodulator of NMDA-receptor-dependent glutamatergic neurotransmission. This function may proceed in an uncontrolled and finally harmful way when abundant t-PA is released secondary to noxious stimuli. Recently it has been demonstrated that active immunization against the t-PA binding site of the NR1 subunit in mice reduces the severity of ischemic and excitotoxic insults in the mouse brain (Benchenane et al., 2007; J. Cell Science120: 578-585). Furthermore, WO 2011/023250 discloses that passive immunization against the N-terminal domain of the NR1 subunit has a neuroprotective effect in an animal model of ischemic stroke.

Treatment of the hemorrhagic stroke as the most common form of intracranial hemorrhage suffers from the additional problem that proper medical treatment of a stroke victim relies on accurate differentiation between ischemic and hemorrhagic stroke. For example, ischemic stroke is typically treated by the administration of tissue plasminogen activator (t-PA) or another "clot busting" agent designed to dissolve clots, thereby restoring blood flow to the brain. However, if t-PA or a similar drug is given to a patient experiencing a hemorrhagic stroke, intracerebral bleeding will be worsened, thereby increasing the severity of the stroke and its consequent damage. In current practice, diagnosis of stroke type is performed by inspection of images obtained by computer tomography (CT) or magnetic resonance imaging (MRI) scans to ascertain or exclude the presence of intracranial hemorrhage. While this technique has been shown to be highly selective if skillfully employed, it requires the availability of expensive and complex CT or MRI equipment, and may be subject to error or uncertainty if the medical personnel conducting and/or interpreting the scan are inexperienced or inadequately trained. Furthermore, the acquisition of a CT or MRI scan is always time consuming and thereby delays the administration of appropriate treatment to the patient. It is therefore advantageous to have a therapeutic intervention which can treat both conditions: ischemic as well as hemorrhagic stroke and thus would not require an accurate diagnostic differentiation prior to initiation.

Thus, it is an objective of the present invention to provide novel means for treating intracranial hemorrhage, in particular ICH.

This objective is solved by providing a medicament for the patient suffering from intracranial hemorrhage comprising a binding protein directed against the amino-terminal domain of the NMDA receptor subunit NR1 (ATD NR1).

It has now been found in a clinically relevant model of intracerebral hemorrhage (ICH) in rats that an intravenous antibody-based therapy targeting the interaction of t-PA with the NR1 subunit of the NMDA receptor exhibits clear histological and functional protective effects after ICH, including reduced neuronal death and inflammatory processes, and better neurological outcomes. Furthermore, the immunotherapy proved to be a safe treatment.

This beneficial efficacy of the α-ATD NR1 antibody was shown in a clinically relevant model of ICH, wherein intrastriatal applied bacterial collagenase disrupts the basal lamina of cerebral blood vessels causing blood to leak into the surrounding brain tissue. This model is widely used in part owing to its simplicity and the fact that this model results in a more consisting hemorrhaging.

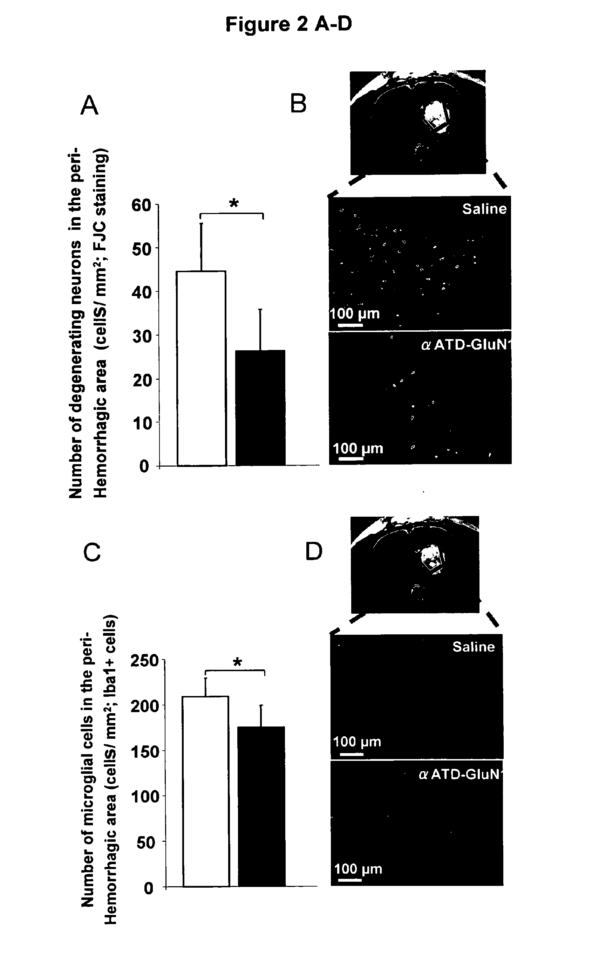

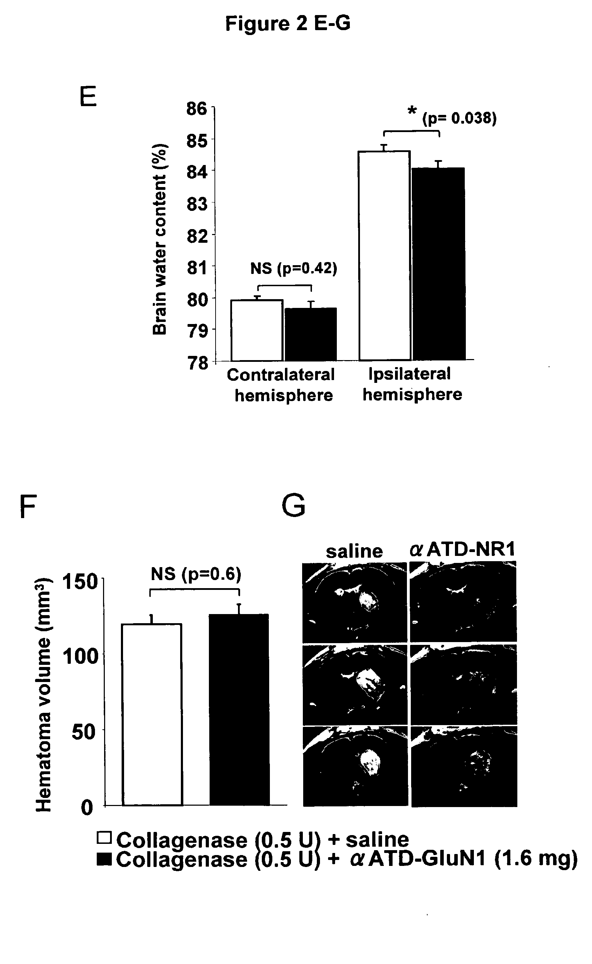

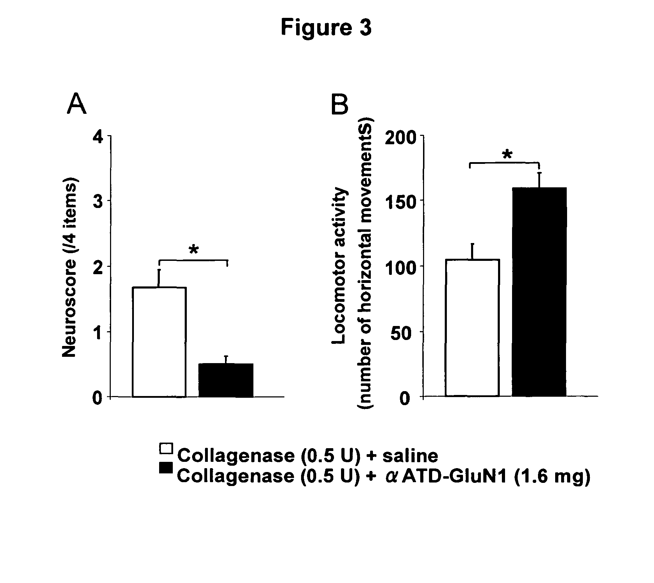

While therapeutic efficacy of the antibodies against detrimental effects of administration of t-PA would be a logical consequence of their design, they most surprisingly were found to be effective without co-administration of t-PA: Rats treated with antibodies in the *in vivo* model of intracerebral hemorrhage displayed strongly decreased brain damage (see Figure 2A) and reduced edema (see Figure 2E). Furthermore, mortality and neurological deficits (see Figure 3) were found to be reduced.

As a result of these findings the ATD NR1 binding protein is also able to inhibit noxious effects of endogenously released t-PA.

In a first aspect of the invention the ATD NR1 binding protein can be used as a monotherapy (i.e. without t-PA co-treatment) for the treatment of intracranial hemorrhage, particularly ICH.

This pathological condition is especially suited for a treatment according the invention since the hemorrhagic condition is accompanied by a disintegration of the blood brain barrier. Hence, the systemically applied binding protein can easily cross the blood brain barrier and reach the affected CNS parenchyma.

Furthermore, the treatment using the binding protein of the invention does not require an accurate diagnostic differentiation between ischemic and hemorrhagic stroke, since said binding protein displays a beneficial effect in ischemic stroke (as disclosed by WO 2011/023250) as well as in hemorrhagic stroke as showed by the inventors herein. For the first time this opens up the possibility for an immediate treatment of the affected subjects which can be treated before diagnosis or even before admission to the hospital. It has to be emphasized that a timely treatment is essential for a successful therapy of cerebrovascular diseases. This is reflected by the catchphrase "Time is brain" which represents a general call to speedy action in acute stroke care.

The binding protein of the invention is directed only against a small part of one of the NMDA subunits. This allows a specific intervention at the NMDA receptor that does not interfere with the normal function of the NMDA receptor but only inhibits the t-PA-induced potentiation of the NMDA activity.

Due to the fact that the binding protein is not directed against t-PA the respective plasminogen activator is not affected in its normal thrombolytic function that is mandatory for a beneficial activity in hemorrhage lysis.

The ATD NR1 binding protein can be used for passive immunization of patients that are in risk of a intracranial hemorrhage or suffer from an intracranial hemorrhage. In contrast to active immunization the blocking effect at the NMDA receptor is immediately achieved which is of high importance since for hemorrhage a timely treatment is a hallmark for a successful outcome.

Since the interplay between t-PA and glutamate (which is the physiological neurotransmitter acting by the NMDA receptor) is critical to synaptic remodeling and plasticity underlying memory and learning processes, the risk of corresponding side effects of treatment modalities affecting their function is apparent. Accordingly, the active ATD-NR1 immunization in mice was reported to impair spatial memory (Benchenane et al., 2007). For the passive immunization as used herein, alterations of cognitive functions including spatial memory, contextual and fear conditioning were not observed. This supports a superior safety profile for this strategy of treating hemorrhage.

### Combination therapy

Since the neuroprotective, anti-inflammatory and anti-edematous effect of the binding protein of the invention allows the safe concomitant thrombolysis using a plasminogen activator, the invention relates further to a method for the treatment of intracranial hemorrhage, particularly ICH, whereby the patient is treated with a therapeutically effective amount of the ATD NR1 binding protein in combination with an effective amount of a plasminogen activator, in particular t-PA, especially when injected into the hematome in order to dissolve it. The invention further relates to a composite comprising t-PA and an antibody directed against the N-terminal domain of the NMDA receptor subunit NR1.

Thus, in one aspect of the invention the ATD NR1 binding protein is used in combination with a medicament comprising a thrombolytic drug, preferably a plasminogen activator. The ATD NR1 binding protein and the plasminogen activator can either be administered simultaneously as one single preparation or simultaneously or sequentially when contained in two separate preparations. The term "sequentially" within the scope of the invention refers to any method of administering the components which does not take place simultaneously. By simultaneous administration is meant the method of administration in which at least one component is administered for example by infusion over a longer period and the other component is also used during this period. If the two components are both administered by infusion over a longer period of time simultaneously means that the infusion periods overlap at least a short time.

### Description of the plasminogen activator

In a further embodiment of the invention the plasminogen activator which is used in combination with the ATD NR1 binding protein is a plasminogen activator selected from the group consisting of recombinant t-PA (rt-PA, e.g. Alteplase) or variants of t-PA such as Pamiteplase, Lanoteplase, Reteplase, Tenecteplase or Monteplase, Urokinase or DSPA variants such as DSPA alpha 1, DSPA alpha 2, DSPA beta or DSPA gamma.

In a preferred embodiment of the invention, the plasminogen activator used for combination treatment of intracranial hemorrhage is a non-neurotoxic plasminogen activator, i.e. a plasminogen activator, which *per se* lacks the potential to activate the NMDA type glutamate receptor. This plasminogen activator favourably is essentially non-activatable by beta-amyloid or prion protein and in the presence of fibrin shows an enhanced activity of more than 550 fold, preferred more than 5,500 fold, most preferred more than 10,000 fold, compared to the activity in the absence of fibrin. In a particular preferred embodiment the increase of activity of the plasminogen activator in the presence of fibrin compared to its activity in the absence of fibrin is more than 100,000. Since the increase in activity of rt-PA is 550, the particularly preferred plasminogen activators for the use according to the invention have an approximately 180-200 fold higher fibrin specificity/selectivity compared to rt-PA.

In a further preferred embodiment of the invention said non-neurotoxic plasminogen activator is derived from the vampire bat *Desmodus rotundus* which is designated as **D**esmodus **R**otundus **S**alivary **P**lasminogen **A**ctivator (DSPA), and which comprises DSPA variants such as DSPA alpha 1, DSPA alpha 2, DSPA beta or DSPA gamma.

In a specific embodiment of the invention said non-neurotoxic plasminogen activator is DSPA alpha1 (desmoteplase).

According to the invention the plasminogen activators can be administered systemically by intravenous injection or locally. For the latter, in an advantageous embodiment of the invention, the PA is locally administered after a puncture of the hematoma, preferably guided by stereotactic methodology. As for the finding of a human dose, the amount of plasminogen activator to be applied can be determined by the size of the hemorrhage as determined by appropriate imaging methods, in a way that the volume of the hemorrhage defines the amount of the plasminogen activator, e.g. DSPA alpha 1, to be administered. In a preferred embodiment the dosage of plasminogen activator such as DSPA alpha 1 in mg equals to the hematoma size in cm³ as determined by T2*-weighted MRI.

### Description of the antibody

In one aspect the invention provides a binding protein comprising a variable heavy chain polypeptide and a variable light chain polypeptide; wherein said binding protein is capable of binding the N-terminal domain of the human NMDA receptor subunit NR1.

In the context of the invention the binding protein is directed against the N-terminal region of the NR1 subunit encoding the amino acids from 19 to 490, preferably encoded by the nucleotide sequence of SEQ ID NO:3 or 4 or encoding amino acids 19 to 371 as disclosed in SEQ ID NO:1 or 2 or a fragment thereof comprising of 8 to 30, preferably 10 to 20 contiguous amino acids wherein the binding protein which is directed against said fragment prevents the cleavage of the NR1 subunit by t-PA.

In one aspect of the invention, the NR1 polypeptide against which the binding protein of the invention is directed, comprises or consists of the amino acid sequence of SEQ ID NO:1 or SEQ ID NO:2 as follows:

SEQ ID NO:1 (rat NR1-1a, NP_058706.1, as 19 to 371)
RAACDPKIVNIGAVLSTRKHEQMFREAVNQANKRHGSWKIQLNATSVTHKPNAIQMALSVC
EDLlSSQVYAILVSHPPTPNDHFTPTPVSYTAGFYRIPVLGLTTRMSIYSDKSIHLSFLRT
VPPYSHQSSVWFEMMRVYNWNHIILLVSDDHEGRAAQKRLETLLEERESKAEKVLQFDPGT
KNVTALLMEARELEARVIILSASEDDAATVYRAAAMLNMTGSGYVWLVGEREISGNALRYA
PDGIIGLQLlNGKNESAHISDAVGWAQAVHELLEKENITDPPRGCVGNTNIWKTGPLFKRV
LMSSKYADGVTGRVEFNEDGDRKFANYSIMNLQNRKLVQVGIYNGTH

SEQ ID NO:2 (human NR1-1, NP_000823.4, as 19 to 371)
RAACDPKIVNIGAVLSTRKHEQMFREAVNQANKRHGSWKIQLNATSVTHKPNAIQMALSVC
EDLlSSQVYAILVSHPPTPNDHFTPTPVSYTAGFYRIPVLGLTTRMSIYSDKSIHLSFLRT
VPPYSHQSSVWFEMMRVYSWNHIILLVSDDHEGRAAQKRLETLLEERESKAEKVLQFDPGT
KNVTALLMEARELEARVIILSASEDDAATVYRAAAMLNMTGSGYVWLVGEREISGNALRYA
PDGIIGLQUNGKNESAHISDAVGWAQAVHELLEKENITDPPRGCVGNTNIWKTGPLFKRVL
MSSKYADGVTGRVEFNEDGDRKFANYSIMNLQNRKLVQVGIYNGTH

In another aspect of the invention, the NR1 polypeptide against which the binding protein of the invention is directed, is encoded by a nucleotide sequence which comprises or consists of the nucleotide sequence of SEQ ID NO:3 or SEQ ID NO:4.

In a further aspect of the invention, the NR1 polypeptide against which the binding protein of the invention is directed, is encoded by a nucleotide sequence which comprises or consists of a nucleic acid molecule that specifically hybridizes to the complement of the nucleic acid molecule of SEQ ID NO: 3 or or SEQ ID NO: 4 under conditions of high stringency, where the hybridisation is performed in 5x SSPE, 5 x Denhardt's solution and 0.5% SDS overnight at 55 to 60°C.

According to the invention the binding protin prevents cleavage of the extracellular domain of the NR1 subunit by a protease, preferably by t-PA and most preferably at the amino acid residue Arg260.

In a further aspect said binding protein is an immunoglobulin molecule, a disulfide linked Fv, a monoclonal antibody, a scFv, a chimeric antibody, a single domain antibody, a CDR-grafted antibody, a diabody, a humanized antibody, a multispecific antibody, a Fab, a dual specific antibody, a dual-variable domain IgG (DVD), a Fab', a bispecific antibody, a F(ab')2, or a Fv.

In another aspect, the binding protein described above comprises a heavy chain immunoglobulin constant domain selected from the group consisting of; a human IgM constant domain, a human IgG4 constant domain, a human IgG1 constant domain, a human IgB constant domain, a human IgG2 constant domain, a human IgG3 constant domain, and a human IgA constant domain.

The binding proteins of the invention are capable of modulating a biological function of the NMDA receptor by binding and preventing the proteolysis of the NMDA receptor. In one aspect, the binding proteins have an on rate constant (Kₒₙ) to said target selected from the group consisting of: at least about 10² M⁻¹s⁻¹; at least about 10³ M⁻¹s⁻¹; at least about 10⁴ M-¹s⁻¹; at least about 10⁵ M⁻¹s⁻¹; and at least about 10⁶ M⁻¹s⁻¹; as measured by surface plasmon resonance. In another aspect, the binding proteins have an off rate constant (K_{off}) to said target selected from the group consisting of: at most about 10⁻³s⁻¹; at most about 10⁻³s⁻¹; at most about 10⁻³s⁻¹; and at most about 10⁻³s⁻¹, as measured by surface plasmon resonance. In another aspect, the binding proteins have a dissociation constant (K_{D}) to said target selected from the group consisting of: at most about 10⁻⁷ M; at most about 10⁻⁸ M; at most about 10⁻⁹ M; at most about 10⁻¹⁰ M; at most about 10⁻¹¹ M; at most about 10⁻¹² M; and at most 10⁻¹³ M.

In one embodiment of the invention the α-ATD NR1 antibody is a monoclonal antibody, preferably a humanized antibody, a single domain antibody or V_{H}H antibody (so called nanobody), a chimeric antibody or deimmunized antibody.

In a preferred embodiment of the invention the binding protein is a human recombinant antibody directed against the N-terminal domain of the human NMDA receptor subunit NR1.

### Treatment

In one aspect of the invention the binding protein is used to treat an intracranial hemorrhage, whereby said hemorrhage might be represented by one of the following pathologies: intra-axial or intracerebral hemorrhage such as intraparenchymal hemorrhage or intraventricular hemorrhage, extra-axial hemorrhage such as epidural hemorrhage, subdural hemorrhage or subarachnoid hemorrhage.

In a preferred embodiment the intraventricular hemorrhage is treated with a binding protein selected from the list consisting of an immunoglobulin molecule, a disulfide linked Fv, a monoclonal antibody, a scFv, a chimeric antibody, a single domain antibody, a CDR-grafted antibody, a diabody, a humanized antibody, a multispecific antibody, a Fab, a dual specific antibody, a dual-variable domain IgG (DVD), a Fab', a bispecific antibody, a F(ab')2, or a Fv.

In a even more preferred embodiment the intraventricular hemorrhage is treated with a monoclonal antibody which is directed against the amino terminal domain of the NR1 subunit of the human NMDA receptor. In particular said monoclonal antibody is a recombinant human or humanized antibody and/or is directed against SEQ ID NO:1 or 2 or a fragment thereof comprising of 8 to 30, preferably 10 to 20 contiguous amino acids.

In a still preferred embodiment the epidural hemorrhage is treated with a binding protein selected from the list consisting of an immunoglobulin molecule, a disulfide linked Fv, a monoclonal antibody, a scFv, a chimeric antibody, a single domain antibody, a CDR-grafted antibody, a diabody, a humanized antibody, a multispecific antibody, a Fab, a dual specific antibody, a dual-variable domain IgG (DVD), a Fab', a bispecific antibody, a F(ab')2, or a Fv.

In a even more preferred embodiment the epidural hemorrhage is treated with a monoclonal antibody which is directed against the amino terminal domain of the NR1 subunit of the human NMDA receptor. In particular said monoclonal antibody is a recombinant human or humanized antibody and/or is directed against SEQ ID NO:1 or 2 or a fragment thereof comprising of 8 to 30, preferably 10 to 20 contiguous amino acids.

In another preferred embodiment the subdural hemorrhage is treated with a binding protein selected from the list consisting of an immunoglobulin molecule, a disulfide linked Fv, a monoclonal antibody, a scFv, a chimeric antibody, a single domain antibody, a CDR-grafted antibody, a diabody, a humanized antibody, a multispecific antibody, a Fab, a dual specific antibody, a dual-variable domain IgG (DVD), a Fab', a bispecific antibody, a F(ab')2, or a Fv.

In a even more preferred embodiment the subdural hemorrhage is treated with a monoclonal antibody which is directed against the amino terminal domain of the NR1 subunit of the human NMDA receptor. In particular said monoclonal antibody is a recombinant human or humanized antibody and/or is directed against SEQ ID NO:1 or 2 or a fragment thereof comprising of 8 to 30, preferably 10 to 20 contiguous amino acids.

In another preferred embodiment the subarachnoid hemorrhage is treated with a binding protein selected from the list consisting of an immunoglobulin molecule, a disulfide linked Fv, a monoclonal antibody, a scFv, a chimeric antibody, a single domain antibody, a CDR-grafted antibody, a diabody, a humanized antibody, a multispecific antibody, a Fab, a dual specific antibody, a dual-variable domain IgG (DVD), a Fab', a bispecific antibody, a F(ab')2, or a Fv.

In a even more preferred embodiment the subarachnoid hemorrhage is treated with a monoclonal antibody which is directed against the amino terminal domain of the NR1 subunit of the human NMDA receptor. In particular said monoclonal antibody is a recombinant human or humanized antibody and/or is directed against SEQ ID NO:1 or 2 or a fragment thereof comprising of 8 to 30, preferably 10 to 20 contiguous amino acids.

In a most preferred embodiment the intraparenchymal hemorrhage is treated with a binding protein selected from the list consisting of an immunoglobulin molecule, a disulfide linked Fv, a monoclonal antibody, a scFv, a chimeric antibody, a single domain antibody, a CDR-grafted antibody, a diabody, a humanized antibody, a multispecific antibody, a Fab, a dual specific antibody, a dual-variable domain IgG (DVD), a Fab', a bispecific antibody, a F(ab')2, or a Fv.

In a even more preferred embodiment the intraparenchymal hemorrhage is treated with a monoclonal antibody which is directed against the amino terminal domain of the NR1 subunit of the human NMDA receptor. In particular said monoclonal antibody is a recombinant human or humanized antibody and/or is directed against SEQ ID NO:1 or 2 or a fragment thereof comprising of 8 to 30, preferably 10 to 20 contiguous amino acids.

Due to the specific and safe use the binding protein of the invention can also be administered as a prophylactic treatment, particularly to a subject having an increased risk for intracranial hemorrhage, which in particular is a intracerebral hemorrhage.

In a further aspect of the invention the increased risk might be due to a surgical procedure which is associated with a recognizable risk of hemorrhage. Examples for said surgical treatment includes vascular neurosurgery such as surgical treatment of unruptured cerebral aneurysms or stereotactic radiosurgery of cerebral arteriovenous malformations, heart surgery, carotid endarterectomy or surgery to other major arteries supplying the central nervous system.

In a preferred embodiment of the invention the binding protein directed against ADT NR1 is administered to the patient before the initiation of the surgical procedure.

In a further aspect of the invention the increased risk for intracranial hemorrhage which qualifies for a treatment of prophylaxis comprising the ATD NR1 binding protein is due to medicative treatment, whereby the medicaments are preferably selected from the group consisting of anticoagulants, antiplatelets, non steroidal anti inflammatory drugs (NSAIDS) and HMG-CoA reductase inhibitors.

### Time window

Due to its safety and the general use for treatment of ischemic and hemorrhagic conditions the anti-ATD NR1 binding protein can be administered to the subject before admission into hospital or diagnosis of hemorrhage.

In a specific embodiment of the invention the ATD NR1-binding protein is administered to the subject after the onset of stroke but before diagnosis of ischemic versus hemorrhagic stroke.

In a further embodiment of the invention the ATD NR1 binding protein can be used as rescue medication in cerebrovascular emergency situations.

In a preferred embodiment the rescue medication is administered by injection whereby the medication is preferably provided as a prefilled syringe, more preferably in form of a pen.

When used as a rescue medication the physician in charge should be experienced in emergency medicine.

As a rescue medication the subject who is in risk of a intracranial hemorrhage should known, should always carry the necessary emergency medicine with him/her.

The invention also provides a pharmaceutical composition comprising the binding protein of the invention and at least one pharmaceutically acceptable carrier or excipient.

In one aspect the invention provides a method of treating an intracranial hemorrhage, in particular ICH, comprising administering a therapeutically effective amount of an isolated binding protein of the invention with or without a therapeutically active amount of a thrombotic drug, preferably a plasminogen activator, more preferably t-PA, most preferably DSPA alpha 1.

### DEFINITIONS

The term "hemorrhage" which is synonymously to the term "bleeding" is defined as a blood loss from the circulatory system.

According to the invention the term "intracranial hemorrhage" is defined as a bleeding within the skull and thus encompasses intra-axial and extra-axial hemorrhages. Intra-axial hemorrhage which is synonymous with the term "intracerebral hemorrhage" includes intraparenchymal hemorrhage and intraventricular hemorrhage. Extra-axial hemorrhage falls into three subtypes, epidural hemorrhage, subdural hemorrhage and subarachnoid hemorrhage.

The term "medicament" is defined as at least one pharmaceutical composition comprising at least one therapeutically effective drug. In one embodiment the medicament may comprise an effective amount of a ATD NR1 binding protein which prevents the plasminogen activator from activating the NMDA glutamate receptor within the patient. When used together with a plasminogen activator, the binding protein can be comprised within the same preparation as the plasminogen activator or can be contained in a separate preparation.

The term "treating" or "treatment" refers to any medical measure for preventing, reducing, mitigating or curing physiological disorders within a patient in the need thereof.

A "therapeutically effective amount" is defined as the amount of active ingredient that will reduce the symptoms and/or improve the outcome associated with a hemorrhage, such as ICH. "Therapeutically effective" also refers to any improvement in disorder severity or the frequency of incidences compared to no treatment. The term "treatment" encompasses either curing or healing as well as mitigation, remission or prevention.

The term "N-terminal domain of the NMDA receptor subunit NR1" (ATD NR1) as used in the context of the present invention is defined as the region of the NR1 subunit of the NMDA receptor that interacts with t-PA. This encompasses NMDA receptors of different species like mouse, rat, pig, bovine, cat, dog, or monkey. Preferably the human NMDA receptor is used. Different isoforms of the NR1 subunits are also included in this definition, preferably the isoforms generated by alternative splicing and including the isoforms NR1-1a, NR1-1b, NR1-2a, NR1-2b, NR1-3a, NR1-3b, NR1-4a, NR1-4b. Preferably the region of the NR1-1a subunit encoding the amino acids 19 to 371 (see SEQ ID:NO. 1) is used.

The term "multivalent binding protein" is used throughout this specification to denote a binding protein comprising two or more antigen binding sites. In an embodiment, the multivalent binding protein is engineered to have three or more antigen binding sites, and is generally not a naturally occurring antibody. The term "multispecific binding protein" refers to a binding protein capable of binding two or more related or unrelated targets. Dual variable domain (DVD) binding proteins as described in WO2010/087972 (the disclosure of which is incorporated herein by reference) comprise two or more antigen binding sites and are tetravalent or multivalent binding proteins. DVDs may be monospecific, i.e., capable of binding one antigen or multispecific, i.e. capable of binding two or more antigens. DVD binding proteins comprising two heavy chain DVD polypeptides and two light chain DVD polypeptides are referred to as DVD-1g. Each half of a DVD-Ig comprises a heavy chain DVD polypeptide, and a light chain DVD polypeptide, and two antigen binding sites. Each binding site comprises a heavy chain variable domain and a light chain variable domain with a total of 6 CDRs involved in antigen binding per antigen binding site.

The term "antibody", as used herein, broadly refers to any immunoglobulin (Ig) molecule comprised of four polypeptide chains, two heavy (H) chains and two light (L) chains, or any functional fragment, mutant, variant, or derivation thereof, which retains the essential epitope binding features of an Ig molecule. Such mutant, variant, or derivative antibody formats are known in the art, non-limiting embodiments of which are discussed below.

In a full-length antibody, each heavy chain is comprised of a heavy chain variable region (abbreviated herein as HCVR or VH) and a heavy chain constant region. The heavy chain constant region is comprised of three domains, CH1, CH2 and CH3. Each light chain is comprised of a light chain variable region (abbreviated herein as LCVR or VL) and a light chain constant region. The light chain constant region is comprised of one domain, CL. The VH and VL regions can be further subdivided into regions of hyper variability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. Immunoglobulin molecules can be of any type (e.g., IgG, IgE, IgM, IgD, IgA and IgY), class (e.g., IgG1, IgG2, IgG3, IgG4, IgA1 and IgA2) or subclass.

The term "antigen-binding portion" of an antibody (or simply "antibody portion"), as used herein, refers to one or more fragments of an antibody that retain the ability to specifically bind to an antigen (e.g., NMDA receptor). It has been shown that the antigen-binding function of an antibody can be performed by fragments of a full-length antibody. Such antibody embodiments may also be bispecific, dual specific, or multi-specific formats; specifically binding to two or more different antigens. Examples of binding fragments encompassed within the term "antigen-binding portion" of an antibody include (i) a Fab fragment, a monovalent fragment consisting of the VL, VH, CL and CHI domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Winter et al., PCT publication WO 90/05144 A1 herein incorporated by reference), which comprises a single variable domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Huston et al. (1988) Proc. Natl. Acad. Sei. USA 85:5879-5883). Such single chain antibodies are also intended to be encompassed within the term "antigen-binding portion" of an antibody. Other forms of single chain antibodies, such as diabodies are also encompassed. Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites. Such antibody binding portions are known in the art (Kontermann and Dubel eds., Antibody Engineering (2001) Springer-Verlag New York. 790 pp. (ISBN 3-540-41354-5).

Still further, an antibody or antigen-binding portion thereof may be part of a larger immunoadhesion molecule, formed by covalent or non covalent association of the antibody or antibody portion with one or more other proteins or peptides. Examples of such immunoadhesion molecules include use of the streptavidin core region to make a tetrameric scFv molecule and use of a cysteine residue, a marker peptide and a C-terminal polyhistidine tag to make bivalent and biotinylated scFv molecules. Antibody portions, such as Fab and F(ab)₂ fragments, can be prepared from whole antibodies using conventional techniques, such as papain or pepsin digestion, respectively, of whole antibodies. Moreover, antibodies, antibody portions and immunoadhesion molecules can be obtained using standard recombinant DNA techniques, as described herein.

The terms "monoclonal antibody" or "monoclonal antibody composition" as used herein refer to a preparation of antibody molecules of a single amino acid composition. Accordingly, the term "human monoclonal antibody" refers to antibodies displaying a single binding specificity which have variable and constant regions derived from human germline immunoglobulin sequences. In one embodiment, the human monoclonal antibodies are produced by a hybridoma which includes a B cell obtained from a transgenic non-human animal, e.g. a transgenic mouse, having a genome comprising a human heavy chain transgene and a light human chain transgene fused to an immortalized cell. Preferably said type anti-ATD NR1 antibody is a monoclonal antibody.

The term "chimeric antibody" refers to a monoclonal antibody comprising a variable region, i.e., binding region, from one source or species and at least a portion of a constant region derived from a different source or species, usually prepared by recombinant DNA techniques. Chimeric antibodies comprising a murine variable region and a human constant region are especially preferred. Such murine/human chimeric antibodies are the product of expressed immunoglobulin genes comprising DNA segments encoding murine immunoglobulin variable regions and DNA segments encoding human immunoglobulin constant regions. Other forms of "chimeric antibodies" encompassed by the present invention are those in which the class or subclass has been modified or changed from that of the original antibody. Such "chimeric" antibodies are also referred to as "class-switched antibodies." Methods for producing chimeric antibodies involve conventional recombinant DNA and gene transfection techniques now well known in the art. See, e.g., US 5,202,238 and US 5,204,244.

The term "humanized antibody" refers to antibodies in which the framework or "complementarity determining regions" (CDR) have been modified to comprise the CDR of an immunoglobulin of different specificity as compared to that of the parent immunoglobulin. In a preferred embodiment, a murine CDR is grafted into the framework region of a human antibody to prepare the "humanized antibody Particularly preferred CDRs correspond to those representing sequences recognizing the antigens noted above for chimeric and bifunctional antibodies.

The term "human antibody", as used herein, is intended to include antibodies having variable and constant regions derived from human germline immunoglobulin sequences. Human antibodies are well-known in the state of the art (van Dijk, M.A., and van de Winkel, J. G., Curr. Opin. Pharmacol. 5 (2001) 368-374). Based on such technology, human antibodies against a great variety of targets can be produced. Examples of human antibodies are for example described in Kellermann, S. A., et al., Curr Opin Biotechnol. 13 (2002) 593-597.

The term "recombinant human antibody", as used herein, is intended to include all human antibodies that are prepared, expressed, created or isolated by recombinant means, such as antibodies isolated from a host cell such as a NS0, NS1, HEK293 or CHO cell or from an animal (e.g. a mouse) that is transgenic for human immunoglobulin genes or antibodies expressed using a recombinant expression vector transfected into a host cell. Such recombinant human antibodies have variable and constant regions derived from human germline immunoglobulin sequences in a rearranged form.

The term "carrier" applied to pharmaceutical compositions of the invention refers to a diluent, excipient, or vehicle with which the therapeutically active compounds of the invention is administered. Such pharmaceutical carriers can be sterile liquids, such as water, saline solutions, aqueous dextrose solutions, aqueous glycerol solutions, and oils, including those of petroleum, animal, vegetable or synthetic origin, such as peanut oil, soybean oil, mineral oil, sesame oil and the like. Suitable pharmaceutical carriers are described in "Remington The Science and Practice of Pharmacy," 21th edition, (David B. Troy ed., 2006, p. 745-775, p. 802-836 and p. 837 - 849).

In the context of the present invention a "pharmaceutically acceptable excipient" means an excipient that is useful in preparing a pharmaceutical composition that is generally safe, non-toxic and neither biologically nor otherwise undesirable, and includes an excipient that is acceptable for veterinary use as well as human pharmaceutical use. A "pharmaceutically acceptable excipient" as used in the present application includes both one and more than one such excipient.

Rescue medication which is synonymous to emergency medication is herein defined as a medication (ADT NR1 binding protein with or without a further drug) which is taken by subject preclinically for the treatment of intracranial hemorrhage or other symptoms associated with intracranial hemorrhage.

### I. IN VIVO EVALUATION FOR IMMUNOTHERAPY OF ICH

### 1. MATERIAL AND METHODS

### 1.1 Animal model of ICH

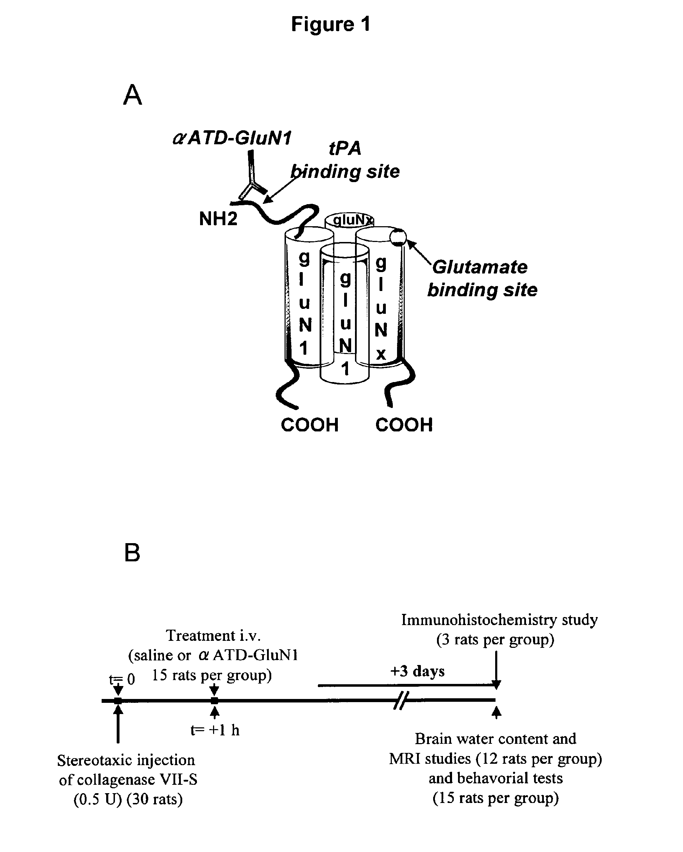

Experiments complied with the European Directives and the French Legislation on Animal Experimentation and were approved by the local ethical committee. ICH was induced by collagenase injection according to Rosenberg et al. 1990. Thirty male Sprague-Dawley rats (250-274 g; CERJ, Paris, France) were anesthetized with isoflurane (1-1.2%) in 70%/30% NO₂/O₂. Using a stereotactic frame, 1 µL of saline containing 0.5 U collagenase VII-S (Sigma, France) was injected through a 30-gauge needle over 5 minutes into the right striatum at bregma: 1.2 mm; mediolateral: 3.5 mm; dorsoventral: 5.3 mm. One hour later, rats were randomized into αATD-GluN1 (15 rats) versus saline groups (15 rats). In each group, all rats were subjected to behavioural assessments, then three rats were used for immunochemistry (Fig 1 B), and the remaining were subjected to MRI and edema measurements. Two animals died in the control group in the 3 days-long period post-ICH.

### 1.2 Passive Immunization with the αATD-GluN1 Polyclonal Antibody

Mouse polyclonal antibodies (αATD-GluN1) were produced and purified as described by Macrez et al., 2011. One hour after collagenase injection, rats received a single injection in the tail vein of either saline (300 µl; saline group) or purified αATD-GluN1 (1.6 mg in 300 µL of saline) (Fig 1 B).

### 1.3 Hemorrhagic Volumes

Magnetic Resonance Imaging (MRI) experiments were performed to evaluate hemorrhagic volumes at 72 hours (n=12 for the αATD-GluN1 group, n=10 for the saline group). MRI was carried out on a 7 Tesla MRI system (Bruker, Germany). During the MRI experiments, anaesthesia was maintained using isoflurane (70%/30% mixture of NO₂/O₂). T2-weighted images were acquired using a RARE sequence: TE/TR 58.2/5200ms. Hemorrhagic volumes were measured using an image analyser (ImageJ 1.45r, NIH). Quantitative analyses were conducted blinded to the treatment.

### 1.4 Brain Water Content

Rats (n=12 for the αATD-GluN1 group, n=10 for the saline group) were euthanized at 72 hours. Brain water content was assessed using standard methods in number of animals (for methodical details see Tejima et al., 2007). A single 4-mm section was cut centered around the haematoma, and edema was calculated as follows: edema= [(wet weight-dry weight)/wet weight]x100. Analyses were performed blindly.

### 1.5 Neurological Tests

All rats were assessed blindly at 72 hours using 2 tests: actimetry and a 5-point neuroscore scale. Neuroscores were graded as follows: 0=no apparent deficit; 1=slight deficit; 2=circling; 3=heavy circling or no movement at all; or 4=death. For actimetry, spontaneous locomotor activity (horizontal movements) was quantified over 10 minutes using activity cages (Imetronic, Pessac, France).

### 1.6 Immunostainings and assessment of neuronal death

Cryostat-cut brain sections were incubated with goat anti-Iba1 polyclonal antibodies (1:800; Abcam, France) before an overnight incubation with the appropriate secondary antibodies to visualise microglial reactivity. Some sections were also stained with Fluorojade C (FJC) as described by Schmued et al. (2005) to investigate neuronal death. All sections were examined using a Leica DM6000 microscope. Images were digitally captured using a coolsnap camera and visualized with Metavue software. Positive cells were quantified using the ImageJ software. Values are the means of 9 serial sections for each animal (n= 3 for the αATD-GluN1 group, n=3 for the saline group).

### 1.6 Statistics

Results are the mean +/- SEM. Statistical analyses were performed using the Kruskall-Wallis test followed by post hoc comparison with the Mann-Whitney U test.

### 2. RESULTS

### 2.1 Immunotherapy with αATD-GluN1 confers neuronal protection and anti-inflammatory effects after ICH

At first, the histological protection of the brain tissue after αATD-GluN1 treatment (single intravenous injection, performed 1 hour after initiation of ICH) in the model of ICH was investigated. Fluorojade stainings performed 3 days after the onset of the injury revealed that the αATD-GluN1 treated animals showed less degenerating neurons surrounding the hematoma compared to untreated animals (-41.1% , n=3, p<0.05; Fig 2A-B). Moreover, the inflammatory response after 3 days was significantly reduced by immunotherapy, as evidenced by the lower number of macrophages/microglia (Iba1 positive cells) surrounding the site of the ICH: 208.9+/-20.1 cells/mm² in the saline group versus 175.0+/-23.9 cells/mm² in the αATD-GluN1 group (-16%; n=3 per group; p<0.05; Fig 2C-D).

### 2.2 αATD-GluN1 reduce ICH-induced brain edema

Edema is associated with a worse clinical outcome in ICH patients. Compared with saline-treated controls, αATD-GluN1 treatment led to a reduced brain edema in the lesioned hemisphere after ICH (water-containing tissue: saline group, 84.5 ± 0.21 %, n=10; αATD-GluN1 group, 83.8 ± 0.23 %; n=12; p=0.038). By contrast, there was no difference between the corresponding contralateral hemispheres (p=0.42) (Fig 2E). Of note, the αATD-GluN1 treatment had no impact on hemorrhagic volumes at day three post-ICH: 119.3 ± 6.2 mm³ in the saline group versus 125.2 ± 6.9 in the αATD-GluN1 group (p=0.6) (Fig 2F-G).

### 2.3 αATD-GluN1 improve global neurological outcome following ICH

αATD-GluN1 treatment significantly improved performance on day 3 post-ICH (saline group, n=13; αATD-GluN1, n=15). The neuro-behavioral score of the αATD-GluN1 treated animals (0.5) was significantly lower than that of saline controls (1.6; p=0.0009; Fig 3A). Consistently, αATD-GluN1 treatment also improved spontaneous activity of the ICH animals (+34.7%, p<0.05; Fig 3B).

### 3. DISCUSSION

Interestingly, the αATD-GluN1 treatment was not only safe, but also offered clear histological and functional protective effects after ICH, including reduced neuronal death and inflammatory processes, and better neurological outcomes.

It is important to note that the αATD-GluN1 antibodies specifically target the noxious interaction of t-PA with the NMDAR, rather than its entire activity profile. Accordingly, these antibodies preserve the beneficial resolving action of t-PA on the hematoma, and at the same time, prevent the neurotoxic sequelae of the t-PA/NMDA receptor interaction. Consistent with this hypothesis, αATD-GluN1 treatment did not influence the size of hematoma, but protected neurons.

Peri-hemorrhagic edema introduces an additional mass effect that may contribute to secondary clinical deterioration and mortality in humans, so that curbing edema formation may be one of the keys to improving the outcome of ICH patients lending. Thus, the finding of the inventors that αATD-GluN1 treatment reduced ICH-induced brain edema as well as mortality and neurological deficits (by 68.7% versus saline) is of high therapeutic interest.

### 4. SUMMARY

Clearly, the αATD-GluN1 antibodies offer the prospect of a safer therapeutic approach compared to unspecific or less specific NMDA receptor antagonists. Side effects due to blocking a neurotransmission pathway largely or entirely are likely to limit or preclude the use of an unspecific or less specific NMDA receptor antagonist in humans, while strategies targeting a modulatory system as provided by the binding proteins of the invention have good chances to be well tolerated.

In conclusion, αATD-GluN1 treatment improves outcome in a rat model of collagenase-induced ICH by inhibiting inflammatory responses, neuronal death and cerebral edema formation. This experimental proof of concept complements the previous evidence that this strategy of immunotherapy is beneficial in ischemic stroke. It suggests that αATD-GluN1 could be administered even before diagnostic measures to distinguish between ischemic and hemorrhagic stroke.

### REFERENCES:

- Benchenane et al., 2007; J Cell Science 120: 578-585
- van Dijk, MA and van de Winkel, JG, 2001, Curr Opin Pharmacol 5: 368-374
- Fernandez-Monreal et al., 2004; J Biol Chem 279: 50850 -50856
- Huston et al., 1988, PNAS 85:5879-5883
- Kellermann, SA et al., 2002, Curr Opin Biotechnol. 13: 593-597
- Kontermann and Dubel eds., Antibody Engineering (2001) Springer-Verlag New York. p. 790ff
- Macrez R et al., Stroke, 2011, 42: 2315-2322
- Rosenberg GA et al. 1990, Stroke 21: 801-807
- Schmued LC et al. 2005, Brain Res 1035: 24-31
- Tejima E et al., J Cereb Blood Flow Metab, 2007, 27: 460-468

**Legend to figures**
- **Figure 1:**: **(A)** Schematic representation of the NMDAR GluN1 subunit (including the recognition site of the ATD-NR1 binding protein, herein an α-ATD NR antibody).
**(B)** Schematic representation of the experimental design.
- **Figure 2**: αATD-GluN1 administration prevents neuronal death, microglial activation, reduces brain edema and has no impact on hemorrhage volume in a model of ICH in mice.
**(A)** Quantification of positive Fluorojade (JFC) cells in the peri-hematoma area 3 days after ICH in control and αATD-GluN1 treated rats (1.6 mg per rat; n=3 per group; *p<0.05 versus saline group; values are mean ± sem).
**(B)** Representative FJC histological staining of neurons collected from brain of controls rats and rats treated with the αATD-GluN1 3 days after ICH. Scale bar: 100 µm.
**(C)** Quantification of macrophages/microglial cells in the peri-hematoma area 3 days after ICH in control and αATD-GluN1 treated rats (1.6 mg per rat; n=3 per group; *p<0.05 versus saline group; values are mean ± sem).
**(D)** Illustrative photomicrographs of immunostainings for microglia (IbA1; red) in the brain of control rats and rats treated with the αATD-GluN1. Scale bar: 100 µm.
**(E)** Bar graphs showing brain water in the contra- and ipsilateral hemispheres 3 days after ICH. There is no difference between the saline and the αATD-GluN1 treated groups in the contralateral hemisphere (p=0.42). For the ipsilateral hemisphere, brain water content is lower in the αATD-GluN1 treated group compared to the saline group (*p<0.05).
**(F)** αATD-GluN1 treatment does not modify the hemorrhagic volumes when compared to the saline group (p=0.6).
**(G)** 3 days after ICH onset, rats were placed in a 7T MRI for T2 brain imaging and determination of the hematoma volume (representative images).
- **Figure 3:**: αATD-GluN1 treatment improves neurological outcome.
**(A)** Neuroscore, *p= 0.0009;
**(B)** locomotor activity, *p< 0.05 both 3 days after ICH. Assays were performed 3 days after ICH.

## Claims

1. A binding protein for the use in the treatment of intracranial hemorrhage, wherein said binding protein is capable of binding to the N-terminal domain of the NMDA receptor subunit NR1 (ATD NR1).

2. The binding protein of claim 1, wherein said binding protein is selected from the group consisting of; an immunoglobulin molecule, a disulfide linked Fv, a monoclonal antibody, a scFv, a chimeric antibody, a single domain antibody, a CDR-grafted antibody, a diabody, a nanobody, a humanized antibody, a multispecific antibody, a Fab, a dual specific antibody, a DVD, a Fab', a bispecific antibody, a F(ab')₂, and a Fv.

3. The binding protein of claim 1 or 2, wherein said NR1 subunit
(i) has the amino acid sequence SEQ ID NO: 1 or 2, and/or
(ii) is encoded by the nucleotide sequences SEQ ID NO: 3 or 4, and/or
(iii) is a nucleic acid molecule that specifically hybridizes to the complement of the nucleic acid molecule of SEQ ID NO: 3 or 4 under conditions of high stringency, where the hybridisation is performed in 5 x SSPE, 5 x Denhardt's solution and 0.5% SDS overnight at 55 to 60°C.

4. The binding protein according to any of the claims 1 to 3, wherein the binding protein is a human recombinant antibody directed against the N-terminal domain of the human NMDA receptor subunit NR1.

5. The binding protein according to one of the above claims, wherein the intracranial hemorrhage is selected from the group of pathologies consisting of; intra-axial or intracerebral hemorrhage such as intraparenchymal hemorrhage or intraventricular hemorrhage, extra-axial hemorrhage such as epidural hemorrhage, subdural hemorrhage or subarachnoid hemorrhage.

6. The binding protein according to one of the above claims, wherein the binding protein is administered as a prophylactic treatment, particularly to a subject having an increased risk for intracranial hemorrhage.

7. The binding protein according to claim 6, wherein the increased risk for intracranial hemorrhage is due to a surgical treatment, particularly selected from the group consisting of, vascular neurosurgery such surgical treatment of unruptured cerebral aneurysms or stereotactic radiosurgery of cerebral arteriovenous malformations, heart surgery, carotid endarterectomy or surgery to other major arteries supplying the central nervous system.

8. The binding protein according to claim 7, wherein the binding protein is administered to the patient before the initiation of the surgical procedure.

9. The binding protein according to claim 6, wherein the increased risk for intracranial hemorrhage is due to medicative treatment, whereby the medicaments are preferably selected from the group consisting of anticoagulants, antiplatelets, non steroidal anti inflammatory drugs (NSAIDS) and HMG-CoA reductase inhibitors.

10. The binding protein according to one of the claims 1 to 4, wherein the binding protein is administered to the subject before admission into hospital or diagnosis of hemorrhage.

11. The binding protein according to one of the claims 1 to 4, wherein the binding protein is administered to the subject after the onset of stroke but before diagnosis of ischemic versus hemorrhagic stroke.

12. The binding protein according to one of the above claims, in combination with a medicament comprising a thrombolytic drug, preferably a plasminogen activator.

13. The binding protein according to claim 12, wherein the thrombolytic drug is a plasminogen activator selected from the group consisting of recombinant t-PA (rt-PA, e.g. Alteplase) or variants of t-PA such as Pamiteplase, Lanoteplase, Reteplase, Tenecteplase or Monteplase, Urokinase or DSPA variants such as DSPA alpha 1, DSPA alpha 2, DSPA beta or DSPA gamma.

14. The binding protein according to one of the claims 1 to 4 for use as injectable rescue medication in cerebrovascular emergency situations.

15. A pharmaceutical composition comprising the binding protein according to one of the claims 1 to 4 and at least one pharmaceutically acceptable carrier or excipient.

16. Method of treating an intracranial hemorrhage, in particular ICH, comprising administering a therapeutically effective amount of an isolated binding protein according to one of the claims 1 to 4 with or without a therapeutically effective amount of a thrombotic drug, preferably a plasminogen activator, more preferably DSPA according to one of the claims 12 and 13.


SEQUENCE LISTING

<110>  PAION Deutschland GmbH
       INSERM

<120>  Immunotherapy for intracranial hemorrhage

<130>  53 589 K

<160>  4

<170>  PatentIn version 3.5

<210>  1
<211>  353
<212>  PRT
<213>  Rattus norvegicus


<220>
<221>  N-terminal sequence without signal peptide
<222>  (1)..(353)
<223>  as 19 to 371 of rat NR1-1a (NP_058706.1)

<400>  1

Arg Ala Ala Cys Asp Pro Lys Ile Val Asn Ile Gly Ala Val Leu Ser
1               5                   10                  15


Thr Arg Lys His Glu Gln Met Phe Arg Glu Ala Val Asn Gln Ala Asn
            20                  25                  30


Lys Arg His Gly Ser Trp Lys Ile Gln Leu Asn Ala Thr Ser Val Thr
        35                  40                  45


His Lys Pro Asn Ala Ile Gln Met Ala Leu Ser Val Cys Glu Asp Leu
    50                  55                  60


Ile Ser Ser Gln Val Tyr Ala Ile Leu Val Ser His Pro Pro Thr Pro
65                  70                  75                  80


Asn Asp His Phe Thr Pro Thr Pro Val Ser Tyr Thr Ala Gly Phe Tyr
                85                  90                  95


Arg Ile Pro Val Leu Gly Leu Thr Thr Arg Met Ser Ile Tyr Ser Asp
            100                 105                 110


Lys Ser Ile His Leu Ser Phe Leu Arg Thr Val Pro Pro Tyr Ser His
        115                 120                 125


Gln Ser Ser Val Trp Phe Glu Met Met Arg Val Tyr Asn Trp Asn His
    130                 135                 140


Ile Ile Leu Leu Val Ser Asp Asp His Glu Gly Arg Ala Ala Gln Lys
145                 150                 155                 160


Arg Leu Glu Thr Leu Leu Glu Glu Arg Glu Ser Lys Ala Glu Lys Val
                165                 170                 175

Leu Gln Phe Asp Pro Gly Thr Lys Asn Val Thr Ala Leu Leu Met Glu
            180                 185                 190

Ala Arg Glu Leu Glu Ala Arg Val Ile Ile Leu Ser Ala Ser Glu Asp
        195                 200                 205

Asp Ala Ala Thr Val Tyr Arg Ala Ala Ala Met Leu Asn Met Thr Gly
    210                 215                 220

Ser Gly Tyr Val Trp Leu Val Gly Glu Arg Glu Ile Ser Gly Asn Ala
225                 230                 235                 240

Leu Arg Tyr Ala Pro Asp Gly Ile Ile Gly Leu Gln Leu Ile Asn Gly
                245                 250                 255

Lys Asn Glu Ser Ala His Ile Ser Asp Ala Val Gly Val Val Ala Gln
            260                 265                 270

Ala Val His Glu Leu Leu Glu Lys Glu Asn Ile Thr Asp Pro Pro Arg
        275                 280                 285

Gly Cys Val Gly Asn Thr Asn Ile Trp Lys Thr Gly Pro Leu Phe Lys
    290                 295                 300

Arg Val Leu Met Ser Ser Lys Tyr Ala Asp Gly Val Thr Gly Arg Val
305                 310                 315                 320

Glu Phe Asn Glu Asp Gly Asp Arg Lys Phe Ala Asn Tyr Ser Ile Met
                325                 330                 335

Asn Leu Gln Asn Arg Lys Leu Val Gln Val Gly Ile Tyr Asn Gly Thr
            340                 345                 350

His

<210>  2
<211>  353
<212>  PRT
<213>  homo sapiens

<220>
<221>  N-terminal sequence without sequence peptide
<222>  (1)..(353)
<223>  as 19 to 371 of human  NR1-1 (NP_000823.4)

<400>  2

Arg Ala Ala Cys Asp Pro Lys Ile Val Asn Ile Gly Ala Val Leu Ser
1               5                   10                  15

Thr Arg Lys His Glu Gln Met Phe Arg Glu Ala Val Asn Gln Ala Asn

            20                  25                  30

Lys Arg His Gly Ser Trp Lys Ile Gln Leu Asn Ala Thr Ser Val Thr
        35                  40                  45

His Lys Pro Asn Ala Ile Gln Met Ala Leu Ser Val Cys Glu Asp Leu
    50                  55                  60

Ile Ser Ser Gln Val Tyr Ala Ile Leu Val Ser His Pro Pro Thr Pro
65                  70                  75                  80

Asn Asp His Phe Thr Pro Thr Pro Val Ser Tyr Thr Ala Gly Phe Tyr
                85                  90                  95

Arg Ile Pro Val Leu Gly Leu Thr Thr Arg Met Ser Ile Tyr Ser Asp
            100                 105                 110

Lys Ser Ile His Leu Ser Phe Leu Arg Thr Val Pro Pro Tyr Ser His
        115                 120                 125

Gln Ser Ser Val Trp Phe Glu Met Met Arg Val Tyr Ser Trp Asn His
    130                 135                 140

Ile Ile Leu Leu Val Ser Asp Asp His Glu Gly Arg Ala Ala Gln Lys
145                 150                 155                 160

Arg Leu Glu Thr Leu Leu Glu Glu Arg Glu Ser Lys Ala Glu Lys Val
                165                 170                 175

Leu Gln Phe Asp Pro Gly Thr Lys Asn Val Thr Ala Leu Leu Met Glu
            180                 185                 190

Ala Arg Glu Leu Glu Ala Arg Val Ile Ile Leu Ser Ala Ser Glu Asp
        195                 200                 205

Asp Ala Ala Thr Val Tyr Arg Ala Ala Ala Met Leu Asn Met Thr Gly
    210                 215                 220

Ser Gly Tyr Val Trp Leu Val Gly Glu Arg Glu Ile Ser Gly Asn Ala
225                 230                 235                 240

Leu Arg Tyr Ala Pro Asp Gly Ile Ile Gly Leu Gln Leu Ile Asn Gly
                245                 250                 255

Lys Asn Glu Ser Ala His Ile Ser Asp Ala Val Gly Val Val Ala Gln
            260                 265                 270

Ala Val His Glu Leu Leu Glu Lys Glu Asn Ile Thr Asp Pro Pro Arg
        275                 280                 285

Gly Cys Val Gly Asn Thr Asn Ile Trp Lys Thr Gly Pro Leu Phe Lys

290                 295                 300

Arg Val Leu Met Ser Ser Lys Tyr Ala Asp Gly Val Thr Gly Arg Val
305                 310                 315                 320

Glu Phe Asn Glu Asp Gly Asp Arg Lys Phe Ala Asn Tyr Ser Ile Met
                325                 330                 335

Asn Leu Gln Asn Arg Lys Leu Val Gln Val Gly Ile Tyr Asn Gly Thr
            340                 345                 350

His

<210>  3
<211>  1059
<212>  DNA
<213>  Rattus norvegicus

<220>
<221>  N-terminal_domain_without_signal_sequence
<222>  (1)..(1059)
<223>  nt 320 - 1378 of NM_017010.1| Rattus norvegicus glutamate
       receptor, ionotropic, N-methyl D-aspartate 1 (Grin1), mRNA

<400>  3
cgcgccgcct gcgaccccaa gatcgtcaac atcggcgcgg tgctgagcac gcgcaagcat      60

gaacagatgt tccgcgaggc agtaaaccag gccaataagc gacacggctc ttggaagata     120

cagctcaacg ccacttctgt cacccacaag cccaacgcca tacagatggc cctgtcagtg     180

tgtgaggacc tcatctctag ccaggtctac gctatcctag ttagccaccc gcctactccc     240

aacgaccact tcactcccac ccctgtctcc tacacagctg gcttctacag aatccctgtc     300

ctgggactga ctacccgaat gtccatctac tctgacaaga gtatccacct gagtttcctt     360

cgcacggtgc cgccctactc ccaccagtcc agcgtctggt ttgagatgat gcgagtctac     420

aactggaacc acatcatcct gctggtcagc gacgaccacg agggacgggc agcgcagaag     480

cgcttggaga cgttgctgga ggaacgggag tccaaggcag agaaggtgct gcagtttgac     540

ccaggaacca agaatgtgac ggctctgctg atggaggccc gggaactgga ggcccgggtc     600

atcatccttt ctgcaagcga ggacgacgct gccacagtgt accgcgcagc cgcaatgctg     660

aacatgacgg gctctgggta cgtgtggctg gtcggggaac gcgagatctc tgggaacgcc     720

ctgcgctacg ctcctgatgg catcatcgga cttcagctca tcaatggcaa gaatgagtca     780

gcccacatca gtgacgccgt gggcgtggtg gcacaggcag ttcacgaact cctagagaag     840

gagaatatca ctgacccacc gcggggttgc gtgggcaaca ccaacatctg gaagacagga     900

ccattgttca agagggtgct gatgtcttct aagtatgcgg acggagtgac tggccgtgtg     960

gaattcaatg aggatgggga ccggaagttt gccaactata gtatcatgaa cctgcagaac    1020

cgcaagctgg tgcaagtggg catctacaat ggtacccat                           1059

<210>  4
<211>  1059
<212>  DNA
<213>  Homo sapiens


<220>
<221>  N-terminal_sequence_without_signal_peptide
<222>  (1)..(1059)
<223>  nt 388 - 1442 of NM_000832.5, Homo sapiens glutamate receptor,
       ionotropic, N-methyl D-aspartate 1 (GRIN1), transcript variant
       NR1-1, mRNA

<400>  4
cgtgccgcgt gcgaccccaa gatcgtcaac attggcgcgg tgctgagcac gcggaagcac      60

gagcagatgt tccgcgaggc cgtgaaccag gccaacaagc ggcacggctc ctggaagatt     120

cagctcaatg ccacctccgt cacgcacaag cccaacgcca tccagatggc tctgtcggtg     180

tgcgaggacc tcatctccag ccaggtctac gccatcctag ttagccatcc acctaccccc     240

aacgaccact tcactcccac ccctgtctcc tacacagccg gcttctaccg catacccgtg     300

ctggggctga ccacccgcat gtccatctac tcggacaaga gcatccacct gagcttcctg     360

cgcaccgtgc cgccctactc ccaccagtcc agcgtgtggt ttgagatgat gcgtgtctac     420

agctggaacc acatcatcct gctggtcagc gacgaccacg agggccgggc ggctcagaaa     480

cgcctggaga cgctgctgga ggagcgtgag tccaaggcag agaaggtgct gcagtttgac     540

ccagggacca agaacgtgac ggccctgctg atggaggcga aagagctgga ggcccgggtc     600

atcatccttt ctgccagcga ggacgatgct gccactgtat accgcgcagc cgcgatgctg     660

aacatgacgg gctccgggta cgtgtggctg gtcggcgagc gcgagatctc ggggaacgcc     720

ctgcgctacg ccccagacgg catcctcggg ctgcagctca tcaacggcaa gaacgagtcg     780

gcccacatca gcgacgccgt gggcgtggtg gcccaggccg tgcacgagct cctcgagaag     840

gagaacatca ccgacccgcc gcggggctgc gtgggcaaca ccaacatctg gaagaccggg     900

ccgctcttca agagagtgct gatgtcttcc aagtatgcgg atggggtgac tggtcgcgtg     960

gagttcaatg aggatgggga ccggaagttc gccaactaca gcatcatgaa cctgcagaac    1020

cgcaagctgg tgcaagtggg catctacaat ggcacccac                           1059